# EUROPÄISCHE PATENTSCHRIFT

(11) **EP 0 557 550 B1**
(45) Veröffentlichungstag und Bekanntmachung des Hinweises auf die Patenterteilung: **27.12.1996**
(21) Anmeldenummer: 92103277.7
(22) Anmeldetag: 26.02.1992
(51) Int. Cl.: A61N 1/368

(54) **Frequenzadaptierender Herzschrittmacher**
Rate adaptive cardiac pacemaker
Stimulateur cardiaque à fréquence ajustable

(43) Veröffentlichungstag der Anmeldung: 01.09.1993
(73) Patentinhaber: Pacesetter AB, 171 95 Solna (SE)
(72) Erfinder: Schwede, Anders Lindgren, S-183 40 Täby (SE)
(74) Vertreter: Lettström, Richard Wilhelm

(56) Entgegenhaltungen:
- EP-A- 0 308 536
- EP-A- 0 381 799
- GB-A- 2 070 282
- US-A- 4 462 406
- US-A- 4 702 253
- US-A- 5 036 849

## Beschreibung

Die Erfindung betrifft einen frequenzadaptierenden Herzschrittmacher mit Mitteln zum Erzeugen von Stimulationsimpulsen mit einer steuerbaren Impulsrate, mit einer Elektrodenanordnung mit mindestens zwei im Herzen angeordneten Elektroden, einer die elektrische Impedanz zwischen den beiden Elektroden erfassenden Impedanzmeßeinrichtung und einer Steuereinrichtung zur Steuerung der Impulsrate in Abhängigkeit von dem erfaßten Impedanzsignal.

Ein derartiger aus der EP-A-0 327 292 bekannter Herzschrittmacher weist einen Stimulationsimpulsgenerator und eine Impedanzmeßeinrichtung auf, die beide jeweils über steuerbare Schalter mit zwei voneinander beabstandeten Elektroden an einem in das Herz eines Patienten eingeführten Elektrodenkatheter verbunden sind. In den Stimulationspausen wird mittels der Impedanzmeßeinrichtung die sich sowohl in Abhängigkeit von der Herztätigkeit als auch von der Atmung des Patienten ändernde Impedanz zwischen den beiden Elektroden erfaßt; aus dem so erhaltenen Impedanzsignal werden die mit der Atmung korrelierenden Frequenzanteile herausgefiltert und daraus das Atemminutenvolumen bestimmt, das zur Steuerung der Impulsrate des Stimulationsimpulsgenerators herangezogen wird.

Bei einem ähnlichen, aus der US-A-4 702 253 bekannten Herzschrittmacher sind drei Elektroden an einem einzigen Elektrodenkatheter angeordnet, wobei zwischen einer ersten dieser drei Elektroden und einer von dem Gehäuse des Herzschrittmachers gebildeten Bezugselektrode die Stimulationsimpulse erzeugt werden, zwischen einer zweiten der drei Elektroden und der Bezugselektrode ein konstanter Meßstrom zur Impedanzmessung erzeugt wird und zwischen der dritten Elektrode und der Bezugselektrode der von dem Meßstrom im Herzen hervorgerufene Spannungsabfall als Impedanzsignal erfaßt wird.

Beide bekannte Herzschrittmacher setzen die Verwendung von Elektrodenkathetern mit zwei (bipolar) oder mehreren Elektroden voraus. Derartige Elektrodenkatheter sind im Unterschied zu unipolaren Elektrodenkathetern mit nur einer Elektrode vergleichsweise dick und unflexibel.

Der Erfindung liegt die Aufgabe zugrunde, eine Impedanzmessung zur Steuerung der Stimulationsimpulsrate bei einem Zweikammerherzschrittmacher zu ermöglichen, ohne daß wie bei den bekannten Herzschrittmachern die Verwendung von zwei- oder mehrpoligen Elektrodenkathetern erforderlich ist.

Gemäß der Erfindung wird die Aufgabe dadurch gelöst, daß bei dem Herzschrittmacher der eingangs angegebenen Art eine der beiden Elektroden an einem ersten, atriellen Elektrodenkatheter und die andere Elektrode an einem zweiten, ventrikulären Elektrodenkatheter angeordnet ist und daß die beiden Elektroden an zwei verschiedenen Stimulationsimpulsgeneratoren des als Zweikammerschrittmacher ausgebildeten Herzschrittmachers angeschlossen sind. Bei dem erfindungsgemäßen Herzschrittmacher erfolgt also die Impedanzmessung unter Ausnutzung der bei einem Zweikammerherzschrittmacher von vorneherein vorgesehenen zwei Elektrodenkatheter, so daß kein besonderer zwei- oder mehrpoliger Elektrodenkatheter für die Impedanzmessung vorgesehen werden muß. Im Unterschied zu den beiden oben genannten bekannten Herzschrittmachern, die als Einkammerherzschrittmacher nur für den Anschluß eines einzigen Elektrodenkatheters ausgebildet sind, sind bei einem Zweikammerherzschrittmacher Anschlüsse für zwei Elektrodenkatheter vorgesehen, so daß zur Realisierung der erfindungsgemäßen Impedanzmessung eine Veränderung der Elektrodenanschlußkonfiguration bei dem Herzschrittmacher nicht erforderlich ist.

Gemäß einer vorteilhaften Weiterbildung des erfindungsgemäßen Herzschrittmachers ist vorgesehen, daß die Impedanzmeßeinrichtung eine Strom- oder Spannungsquelle zur Erzeugung eines Meßstroms bzw. einer Meßspannung zwischen einem Ausgangsanschluß und einem von dem Gehäuse des Herzschrittmachers gebildeten Bezugspotentialanschluß aufweist und daß die atrielle Elektrode über eine erste steuerbare Schalteranordnung und die ventrikuläre Elektrode über eine zweite steuerbare Schalteranordnung wahlweise mit dem Ausgangsanschluß oder dem Bezugspotentialanschluß verbindbar sind. Dadurch ist es möglich, den Meßstrom bzw. die Meßspannung wahlweise zwischen beiden Elektroden oder jweils einer der beiden Elektroden und dem Gehäuse des Herzschrittmachers zu erzeugen.

Eine weitere vorteilhafte Weiterbildung des erfindungsgemäßen Herzschrittmachers besteht darin, daß die Impedanzmeßeinrichtung einen Meßverstärker mit zwei Eingangsanschlüssen aufweist, von denen der eine Eingangsanschluß über eine dritte steuerbare Schalteranordnung wahlweise mit der atriellen Elektrode oder der ventrikulären Elektrode verbindbar ist und der andere Eingangsanschluß über eine vierte steuerbare Schalteranordnung wahlweise mit einer der beiden Elektroden oder dem Bezugspotentialanschluß verbindbar ist. Damit ist es möglich, das Impedanzsignal wahlweise zwischen den beiden Elektroden oder jeweils einer der beiden Elektroden und dem Bezugspotentialanschluß zu erfassen.

Im folgenden wird die Erfindung anhand eines in den Figuren der Zeichung dargestellten Ausführungsbeispiels erläutert; im einzelnen zeigen:
- Figur 1: die bei dem erfindungsgemäßen Herzschrittmacher vorgesehene Anordnung der Elektroden im Herzen,
- Figur 2: ein Blockschaltbild des erfindungsgemäßen Herzschrittmachers
- Figur 3: ein Ausführungsbeispiel für die Impedanzmeßeinrichtung des erfindungsgemäßen Herzschrittmachers.

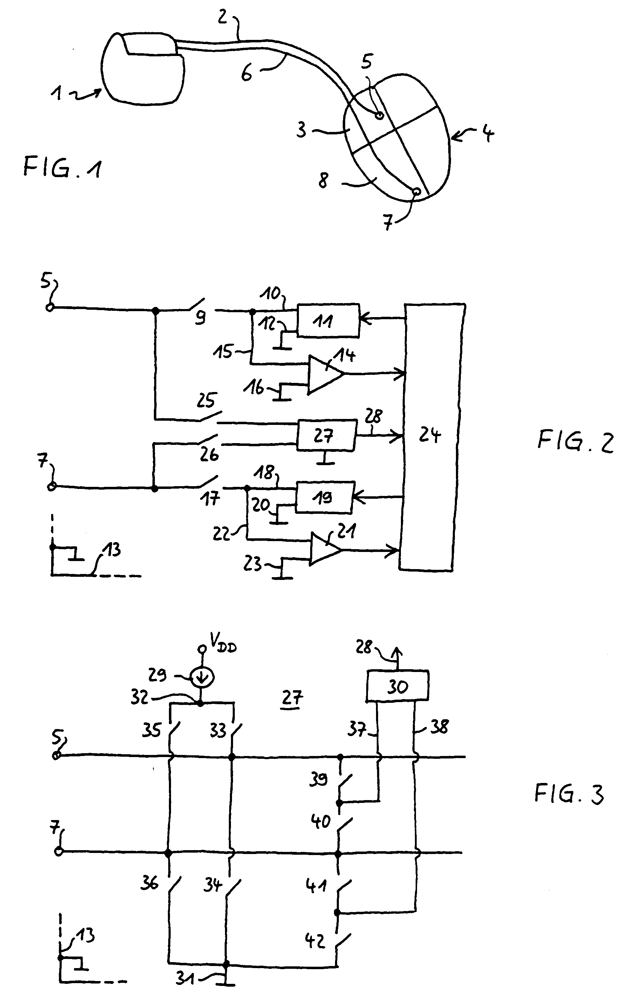

Figur 1 zeigt einen als Zweikammerherzschrittmacher ausgebildeten Herzschrittmacher 1 mit einem ersten, atriellen Elektrodenkatheter 2, der an seinem distalen Ende eine erste, im rechten Atrium 3 des Herzens 4 eines Patienten plazierte Elektrode 5 aufweist, und mit einem zweiten ventrikulären Elektrodenkatheter 6, der mit einer zweiten Elektrode 7 an seinem distalen Ende in den rechten Ventrikel 6 des Herzens 4 eingeführt ist.

Wie das Blockschaltbild in Figur 2 zeigt, ist die atrielle Elektrode 5 über einen steuerbaren Schalter 9 an dem ersten Ausgangsanschluß 10 eines Stimulationsimpulsgenerators 11 angeschlossen, dessen zweiter Ausgangsanschluß 12 potentialmäßig mit dem Gehäuse 13 des Herzschrittmachers 1 verbunden ist. Ein Detektor 14 zur Detektion atrieller Ereignisse ist mit einem ersten Eingangsanschluß 15 an dem Ausgangsanschluß 10 des Stimulationsimpulsgenerators 11 angeschlossen und mit seinem zweiten Eingangsanschluß 16 ebenfalls mit dem Herzschrittmachergehäuse 13 verbunden.

Die ventrikuläre Elektrode 7 ist über einen zweiten steuerbaren Schalter 17 an einem Ausgangsanschluß 18 eines zweiten Stimulationsimpulsgererators 19 angeschlossen, dessen zweiter Ausgangsanschluß 20 mit dem Herzschrittmachergehäuse 13 verbunden ist. Ein weiterer Detektor 21 zur Detektion ventrikulärer Ereignisse ist mit seinem ersten Eingangsanschluß 22 an dem Ausgangsanschluß 18 des zweiten Stimulationsimpulsgenerators 19 angeschlossen und mit seinem zweiten Eingangsanschluß 23 ebenfalls mit dem Herzschrittmachergehäuse 13 verbunden. Die beiden Stimulationsimpulsgeneratoren 11 und 19 und die beiden Detektoren 14 und 21 sind an einer Herzschrittmachersteuerung 24 angeschlossen, die nach jedem stimulierten oder detektierten natürlichen Ereignis im Atrium 3 ein Basiszeitintervall startet und die Abgabe eines Stimulationsimpulses im Atrium durch den atriellen Stimulationsimpulsgenerator 11 auslöst, wenn das Basiszeitintervall ausläuft, ohne daß ein natürliches atrielles Ereignis von dem atriellen Detektor 11 detektiert wurde. Nach jedem stimulierten oder detektierten atriellen Ereignis wird ein atrioventrikuläres Zeitintervall gestartet und die Abgabe eines Stimulationsimpulses im Ventrikel 8 ausgelöst, wenn das Zeitintervall ausläuft, ohne daß ein natürliches Ereignis im Ventrikel 8 von dem Detektor 21 detektiert wurde.

An der atriellen Elektrode 5 und der ventrikulären Elektrode 7 ist ferner über zwei weitere Schalter 25 und 26 eine Impedanzmeßeinrichtung 27 zur Messung der Impedanz des Körpergewebes zwischen den Elektroden 5 und 7 und gegebenenfalls dem Herzschrittmachergehäuse 13 angeschlossen. Die Impedanz stellt dabei einen physiologischen Funktionsparameter dar, der sich sowohl in Abhängigkeit von der Atmung des Patienten als auch in Abhängigkeit von dessen Herztätigkeit ändert. Das Impedanzsignal der Impedanzmeßeinrichtung 27 wird über eine Ausgangsleitung 28 der Herzschrittmachersteuerung 24 zugeführt, in der das Basiszeitintervall nach einer Filterung des Impedanzsignals in Abhängigkeit von der Atmung des Patienten, beispielsweise dem Atemminutenvolumen, oder der Herztätigkeit, beispielsweise dem Herzzeitvolumen, geändert wird.

In Figur 3 ist ein Ausführungsbeispiel für die Impedanzmeßeinrichtung 27 dargestellt, die eine Stromquelle 29 und einen Meßverstärker 30 aufweist. Die Stromquelle 29 wird durch die Versorgungsspannung einer hier nicht gezeigten Batterie des Herzschrittmachers 1 zwischen einem Batteriepotentialanschluß V_{DD} und einem mit dem Gehäuse 13 des Herzschrittmachers 1 verbundenen Bezugspotentialanschluß 31 gespeist und gibt an ihrem Ausgangsanschluß 32 einen konstanten, vorzugsweise gepulsten Strom ab. Die atrielle Elektrode 5 ist über eine erste steuerbare Schalteranordnung bestehend aus einem Schalter 33 und einem Schalter 34 mit dem Ausgangsanschluß 32 der Stromquelle 29 und dem Bezugspotentialanschluß 31 verbunden. Auf entsprechende Weise ist die ventrikuläre Elektrode 7 über eine zweite steuerbare Schalteranordnung bestehend aus den Schaltern 35 und 36 mit dem Ausgangsanschluß 32 der Stromquelle 29 und dem Bezugspotentialanschluß 31 verbunden. Je nachdem, welcher von den Schaltern 33 bis 36 geschlossen ist, fließt der von der Stromquelle 29 abgegebene Strom über die Körperimpedanz zwischen den beiden Elektroden 5 und 7 oder einer der beiden Elektroden 5 und 7 und dem Gehäuse 13 des Herzschrittmachers. Der Meßverstärker 30 weist zwei Eingangsanschlüsse 37 und 38 auf, von denen der mit 37 bezeichnete Eingangsanschluß über eine dritte steuerbare Schalteranordnung bestehend aus den Schaltern 39 und 40 wahlweise mit der atriellen Elektrode 5 oder der ventrikulären Elektrode 7 verbunden ist und der andere Eingangsanschluß 38 über eine vierte steuerbare Schalteranordnung bestehend aus den Schaltern 41 und 42 wahlweise mit der ventrikulären Elektrode 7 oder dem Bezugspotentialanschluß 31 verbunden ist. Alternativ zu dem gezeigten Ausführungsbeispiel ist es auch möglich, daß der zweite Eingangsanschluß 38 über den Schalter 41 anstelle mit der ventrikulären Elektrode 7 mit der atriellen Elektrode 5 verbunden ist. Je nachdem welcher der Schalter 39 bis 42 geöffnet oder geschlossen ist, wird der Meßverstärker 30 eingangsseitig an die Elektroden 5 und 7 oder an eine der Elektroden 5 und 7 und den Bezugspotentialanschluß 31 angeschlossen. Wenn alle Schalter 33 bis 36 und 39 bis 42 geöffnet sind, sind die Stromquelle 29 und der Meßverstärker 30 von den Elektroden 5 und 7 und dem Bezugspotentialanschluß 31 abgekoppelt, so daß sich die Stimulationsimpulsgeneratoren 11 und 19 und die Detektoren 14 und 21 einerseits und die Impedanzmeßeinrichtung 27 andererseits nicht gegenseitig beeinflussen können.

### Bezugszeichenliste

- 1: Herzschrittmacher
- 2: atrieller Katheter
- 3: Atrium
- 4: Herz
- 5: atrielle Elektrode
- 6: ventrikulärer Katheter
- 7: ventrikuläre Elektrode
- 8: Ventrikel
- 9: Schalter
- 10: erster Ausgangsanschluß von 11
- 11: Stimulationsimpulsgenerator
- 12: zweiter Ausganganschluß von 11
- 13: Gehäuse von 1
- 14: Detektor
- 15,16: Eingangsanschlüsse von 14
- 17: Schalter
- 18: erster Ausgangsanschluß von 19
- 19: zweiter Stimulationsimpulsgenerator
- 20: zweiter Ausgangsanschluß von 19
- 21: weiterer Detektor
- 22,23: Eingangsanschlüsse von 21
- 24: Herzschrittmacher
- 25,26: weitere Schalter
- 27: Impedanzmeßeinrichtung
- 28: Ausgangsleitung von 27
- 29: Stromquelle
- 30: Meßverstäker
- 31: Bezugspotentialanschluß
- 32: Ausgangsanschluß von 29
- 33-36: Schalter
- 37,38: Eingangsanschlüsse von 30
- 39-42: Schalter
- V_{DD}: Batteriepotentialanschluß

## Patentansprüche

1. Frequenzadaptierender Herzschrittmacher mit Mitteln (11,19) zum Erzeugen von Stimulationsimpulsen mit einer steuerbaren Impulsrate, mit einer Elektrodenanordnung mit mindestens zwei im Herzen (4) angeordneten Elektroden (5,7), einer die elektrische Impedanz zwischen den beiden Elektroden (5,7) erfassenden Impedanzmeßeinrichtung (27) und einer Steuereinrichtung (24) zur Steuerung der Impulsrate in Abhängigkeit von dem erfaßten Impedanzsignal, **dadurch gekennzeichnet,** daß eine der beiden Elektroden (z.B. 5) an einem ersten, atriellen Elektrodenkatheter (2) und die andere Elektrode (7) an einem zweiten, ventrikulären Elektrodenkatheter (6) angeordnet ist und daß die beiden Elektroden (5,7) an zwei verschiedenen Stimulationsimpulsgeneratoren (11,19) des als Zweikammerschrittmacher ausgebildeten Herzschrittmachers (1) angeschlossen sind.

2. Frequenzadaptierter Herzschrittmacher nach Anspruch 1, **dadurch gekennzeichnet,** daß die Impedanzmeßeinrichung (27) eine Strom- oder Spannungsquelle (29) zur Erzeugung eines Meßstroms bzw. einer Meßspannung zwischen einem Ausgangsanschluß (32) und einem von dem Gehäuse (13) des Herzschrittmachers (1) gebildeten Bezugspotentialanschluß (31) aufweist und daß die atrielle Elektrode (5) über eine erste steuerbare Schalteranordnung (33,34) und die ventrikuläre Elektrode (6) über eine zweite steuerbare Schalteranordnung (35,36) wahlweise mit dem Ausgangsanschluß (32) oder dem Bezugspotentialanschluß (31) verbindbar sind.

3. Frequenzadaptierender Herzschrittmacher nach Anspruch 2, **dadurch gekennzeichnet,** daß die Impedanzmeßeinrichtung (27) einen Meßverstärker (30) mit zwei Eingangsanschlüssen (37,38) aufweist, von denen der eine Eingangsanschluß (37) über eine dritte steuerbare Schalteranordnung (39,40) wahlweise mit der atriellen Elektrode (5) oder der ventrikulären Elektrode (7) verbindbar ist und der andere Eingangsanschluß (38) über eine vierte steuerbare Schalteranordnung (41,42) wahlweise mit einer der beiden Elektroden (5,7) oder dem Bezugspotentialanschluß (31) verbindbar ist.

## Claims

1. Rate adaptive cardiac pacemaker comprising means (11-19) for the production of stimulation pulses with a controllable impulse rate, an electrode arrangement with at least two electrodes (5,7) arranged in the heart (4), an impedance measuring means (27) for measuring the electrical impedance between the two electrodes (5,7) and a control means (24) for controlling the impulse rate as a function of the measured impedance signal, characterised in that one of the two electrodes (for example 5) is mounted on a first atrial electrode catheter (2) and the second electrode (7) is mounted on a second ventricular electrode catheter (6) and the two electrodes (5,7) are connected to two separate stimulation impulse generators (11,19) of the pacemaker (1) which is a dual chamber pacemaker.

2. Rate adaptive cardiac pacemaker according to claim 1, characterised in that the impedance measuring means (27) comprises a current or voltage source (29) which produces respectively a measuring current or a measuring voltage between an output connector (32) and a reference potential connector (31) formed by the casing (13) of the pacemaker (1) and that the atrial electrode (5) by means of a first controllable switching means (33,34) and the ventricular electrode (6) by means of a second controllable switching means (35,36) are selectively connectable with the output connector (32) or the reference potential connector (31).

3. Rate adaptive cardiac pacemaker according to claim 2, characterised in that the impedance measuring means (27) comprises a measuring amplifier (30) with two input connectors (37,38) of which one (37) can be connected by means of a third controllable switching means (39,40) selectively with the atrial electrode (5) or the ventricular electrode (7) and that the other input connector (38) by means of a fourth controllable switching (41,42) is selectively connectable with one of the two electrodes (5,7) or the reference potential connector (31).

## Revendications

1. Stimulateur cardiaque à adaptation de fréquence, comportant des moyens (11,19) de production d'impulsions de stimulation à un taux d'impulsion pouvant être commandé, un dispositif à électrodes comportant au moins deux électrodes (5,7) disposées dans le coeur (4), un dispositif (27) de mesure d'impédance détectant l'impédance électrique entre les deux électrodes (5,7) et un dispositif (24) de commande du taux d'impulsion en fonction du signal d'impédance détecté, caractérisé en ce que l'une des deux électrodes (par exemple 5) est montée sur un premier cathéter (2) auriculaire d'électrodes et l'autre électrode (7) est montée sur un second cathéter (6) ventriculaire d'électrodes et les deux électrodes (5,7) sont connectées à deux générateurs (11,19) différents d'impulsions de stimulation du stimulateur cardiaque (1) constitué en stimulateur à deux chambres.

2. Stimulateur cardiaque à adaptation de fréquence suivant la revendication 1, caractérisé en ce que le dispositif (27) de mesure d'impédance comporte une source (29) de courant ou de tension destinée à la production d'un courant de mesure ou d'une tension de mesure entre une borne (32) de sortie et une borne (31) de potentiel de référence formée par le boîtier (13) du stimulateur cardiaque (1) et l'électrode auriculaire (5) peut être reliée par l'intermédiaire d'un premier dispositif (33,34) à interrupteurs pouvant être commandé et l'électrode ventriculaire (6) peut être reliée par l'intermédiaire d'un second dispositif (35,36) à interrupteurs pouvant être commandé au choix à la borne (32) de sortie ou à la borne (31) de potentiel de référence.

3. Stimulateur cardiaque à adaptation de fréquence suivant la revendication 2, caractérisé en ce que le dispositif (27) de mesure d'impédance comporte un amplificateur (30) de mesure à deux bornes (37,38) d'entrée, dont l'une (37) peut être reliée par l'intermédiaire d'un troisième dispositif (39,40) à interrupteurs pouvant être commandé au choix à l'électrode auriculaire (5) ou à l'électrode ventriculaire (7) et l'autre borne (38) d'entrée peut être reliée par l'intermédiaire d'un quatrième dispositif (41,42) à interrupteurs pouvant être commandé au choix à l'une des deux électrodes (5,7) ou à la borne (31) de potentiel de référence.
